Europäisches Patentamt

European Patent Office

Office européen des brevets

(11) Veröffentlichungsnummer: **0 006 197**
B1

# (12) EUROPÄISCHE PATENTSCHRIFT

(45) Veröffentlichungstag der Patentschrift:
12.01.83

(21) Anmeldenummer: 79101831.0

(22) Anmeldetag: 08.06.79

(51) Int. Cl.³: **G 01 S 7/52**, G 10 K 11/34,
A 61 B 10/00

(54) **Ultraschall-Bildgerät und dessen Verwendung für die medizinische Ultraschall-Diagnostik.**

(30) Priorität: 16.06.78 CH 6587/78

(43) Veröffentlichungstag der Anmeldung:
09.01.80 Patentblatt 80/1

(45) Bekanntmachung des Hinweises auf die Patenterteilung:
12.01.83 Patentblatt 83/2

(84) Benannte Vertragsstaaten:
CH DE FR GB IT NL SE

(56) Entgegenhaltungen:
DE-A-2 651 786
DE-A-2 736 310
DE-A-2 811 544
US-A-3 859 622
US-A-3 869 693

Ultrasonics, Juli 1968, Seiten 153—159

(73) Patentinhaber: **F. HOFFMANN-LA ROCHE & CO.
Aktiengesellschaft, CH-4002 Basel (CH)**

(72) Erfinder: **Burckhardt, Christoph Benedikt, Dr.,
Rebgasse 14, CH-4132 Muttenz (CH)**
Erfinder: **Grandchamp, Pierre-André, Dr., 8 rue de la
Borne Royale, F-78190 Voisins-le-Bretonneux (FR)**
Erfinder: **Hoffmann, Heinz, Dr., Schlossgasse 55,
D-7889 Grenzach (DE)**
Erfinder: **Fehr, Rainer, Ringstrasse 2, CH-4153 Reinach
(CH)**

(74) Vertreter: **Wuesthoff, Franz, Dr.-Ing. et al, Patentanwälte
Wuesthoff -v. Pechmann-Behrens-Goetz
Schweigerstrasse 2, D-8000 München 90 (DE)**

Ultraschall-Bildgerät und dessen Verwendung für die medizinische Ultraschall-Diagnostik

Die Erfindung betrifft ein Ultraschall-Bildgerät mit einer Wandlereinrichtung, die aus einer festen länglichen Reihe Wandlerelemente besteht und mit der ein Ultraschallstrahl durch Umwandlung impulsartiger, von einem Sender abgegebener Sendesignale erzeugt wird, wobei jedem Wandlerelement ein Sendesignal mit einer vorher bestimmten zeitlichen Lage zugeführt wird, sowie die Verwendung des Geräts für die medizinische Ultraschall-Diagnostik.

Für manche Anwendungen der Ultraschalldiagnostik ist es vorteilhaft, wenn der Ultraschall bzw. die Empfangscharakteristik eine Schwenkung ausführt. Diese Drehbewegung ist bei der Untersuchung des Herzens mit Ultraschallwellen besonders wichtig, da für diese Untersuchung die hindernisfreie Fortpflanzung der Ultraschallwellen nur durch ein relativ kleines «Fenster» möglich ist.

Es ist bereits ein Verfahren bzw. ein Gerät der obenerwähnten Art bekannt, bei dem die Schwenkung des Ultraschallstrahls mit einer Anordnung von Schaltern und Verzögerungsgliedern erzeugt wird (J.C.Somer, «Electronic sector scanning for ultrasonic diagnosis», Ultrasonics, Juli 1968, Seiten 153–159), wobei die Öffnung bzw. Schliessung der Schalter elektronisch gesteuert wird.

Die grössten Nachteile dieses bekannten Verfahrens sind die Komplexität der elektronischen Schaltung, und die grosse Anzahl Schalter und Verzögerungsglieder die benötigt werden, um es durchzuführen.

Eine typische Wandleranordnung hat z.B. 32 Elemente. Wenn der Strahl z.B. in 64 verschiedene Richtungen abgelenkt werden soll, so muss das Gerät so gebaut sein, dass das Sende- oder Echosignal von jedem Element um 64 verschiedene Zeiten verzögert werden kann. Im ganzen werden also 32·64=2048 verschiedene Verzögerungswerte benötigt. Die dafür erforderlichen elektronischen Verzögerungselemente und die zugehörigen Schalter lassen ein solches Gerät sehr komplex und teuer werden.

Der Erfindung liegt daher die Aufgabe zugrunde, ein Gerät der eingangs genannten Art zur Verfügung zu stellen, das sich einfach und mit geringem Aufwand herstellen lässt.

Das erfindungsgemässe Gerät ist dadurch gekennzeichnet, dass der Sender enthält:
einen Multiplizierer zur Bildung des Produkts aus einer Impulsfolge der Sendefrequenz und einem Impuls;

einen ersten mit einem Eingang des Multiplizierers verbundenen Multiplexer, an dessen Eingängen eine Mehrzahl von gegeneinander um einen Bruchteil einer Periode des Sendesignals verschobenen Impulsfolgen der Sendefrequenz und vorbestimmter Phasenwinkel vorhanden sind und der auf ein erstes Steuersignal anspricht, um einen ersten Eingang des Multiplizierers wahlweise eine der Impulsfolgen zuzuführen, die einen geeigneten Phasenwinkel hat;

einen zweiten mit einem zweiten Eingang des Multiplizierers verbundenen Multiplexer, an dessen Eingängen eine Mehrzahl von Impulsen gelangen, die in Bezug auf ein Referenzsignal verschiedene Verzögerungen aufweisen und deren Dauer mehrere Impulse einer Impulsfolge der Sendefrequenz umfasst, welcher zweite Multiplexer auf ein zweites Steuersignal anspricht, um einem zweiten Eingang des Multiplizierers wahlweise einen der Impulse zuzuführen, der eine geeignete Verzögerung in Bezug auf das Referenzsignal hat, und

einen mit dem Ausgang des Multiplizierers verbundenen Signalformer, der dazu dient, von dem Ausgangssignal des Multiplizierers einen eine Sinuswelle der Sendefrequenz einhüllenden Sendeimpuls abzuleiten, der einem Wandlerelement der Ultraschallwandleranordnung zugeführt wird.

Gegenstand der Erfindung ist ferner die Verwendung des erfindungsgemässen Geräts für die medizinische Ultraschall-Diagnostik.

Der Hauptvorteil der Erfindung liegt darin, dass sie eine wesentliche Verringerung des Schaltungsaufwands und dadurch die Herstellung von preiswerten Ultraschall-Diagnostikgeräten ermöglicht. Dies trägt dazu bei, die Durchführung von Ultraschalluntersuchungen zu verbilligen, insbesondere im Bereich der medizinischen Ultraschall-Diagnostik.

Nachstehend werden einige Ausführungsbeispiele der Erfindung anhand der beiliegenden Zeichnungen beschrieben. Es zeigen

Fig. 1 eine schematische Darstellung der Wandlerelemente einer bekannten, phasengesteuerten Ultraschallwandleranordnung und des damit erzeugten Ultraschallstrahls,

Fig. 2 ein Blockschaltbild einer erfindungsgemässen Schaltung zur Erzeugung des Sendesignals für ein Wandlerelement,

Fig. 3 einige Signalverläufe zur Erläuterung der Erzeugung eines Sendesignals mit der Schaltung gemäss Fig. 2,

Fig. 4 ein Blockschaltbild eines Ultraschall-Abbildungsgerätes, das die erfindungsgemässe Schaltung gemäss Fig. 2 enthält.

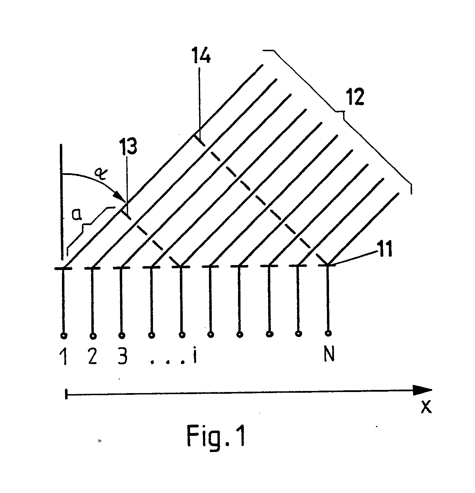

Anhand der Fig. 1 wird nun das Prinzip des bekannten Verfahrens zur elektronischen Schwenkung eines Ultraschallstrahls mit einer sogenannten phasengesteuerten Ultraschallwandleranordnung («ultrasonic phased array») erläutert. Wie in der Fig. 1 gezeigt wird dabei ein Ultraschallwandler verwendet, der in eine Anzahl Wandlerelemente 11 unterteilt ist. Alle Elemente werden für Senden und Empfang benützt. Beim Senden wird die Strahlschwenkung dadurch erreicht, dass man die Elemente mit verschieden verzögerten Impulsen anregt.

Beim Empfang wird die Schwenkung der Empfangscharakteristik dadurch erreicht, dass man die Echosignale, die von den verschiedenen Elementen empfangen werden, verschieden verzögert. Der Schwenkungswinkel des Strahles bzw. der Empfangscharakteristik wird durch diese Verzögerungen bestimmt.

Die Zusammenhänge sind aus Fig. 1 ersichtlich. Hier wird ein Strahl 12 gezeigt, der um den Winkel α nach rechts abgelenkt ist. Die gestrichelten Linien 13 und 14 stellen Wellenfronten dar. Damit beim Senden die Welle vom Element i mit der Welle vom Element 1 in Phase ist, muss sich die Welle vom Element 1 bereits um die Distanz a fortgepflanzt haben, wenn die Welle vom Element i ausgesendet wird, also

$$\frac{a}{x_i} = \sin \alpha,$$

wobei $x_i$ die x-Koordinate des Elementes i ist. Die Strecke a wird von der Welle in der Zeit $\tau_i$ zurückgelegt,

$$a = c\tau_i,$$

wobei c die Schallgeschwindigkeit ist. Beim Senden muss also der Sendeimpuls des Elementes i um

$$\alpha_i = \frac{a}{c} = \frac{x_i \sin \alpha}{c}$$

verzögert werden.

Nach dieser Formel lassen sich die notwendigen Verzögerungen für alle Elemente und Winkel berechnen. Die notwendige Verzögerung des Empfangssignals berechnet sich nach derselben Formel.

Wie in der Beschreibungseinleitung bereits erwähnt, liegt der grösste Nachteil dieses bekannten Verfahrens, in dem grossen Aufwand der Anordnung die benötigt wird, um es durchzuführen.

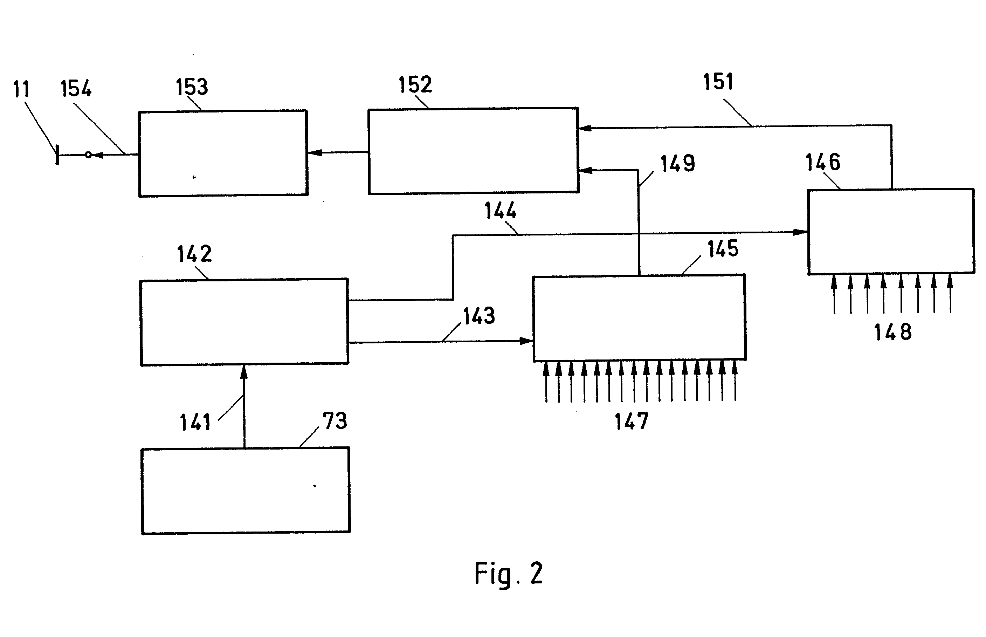

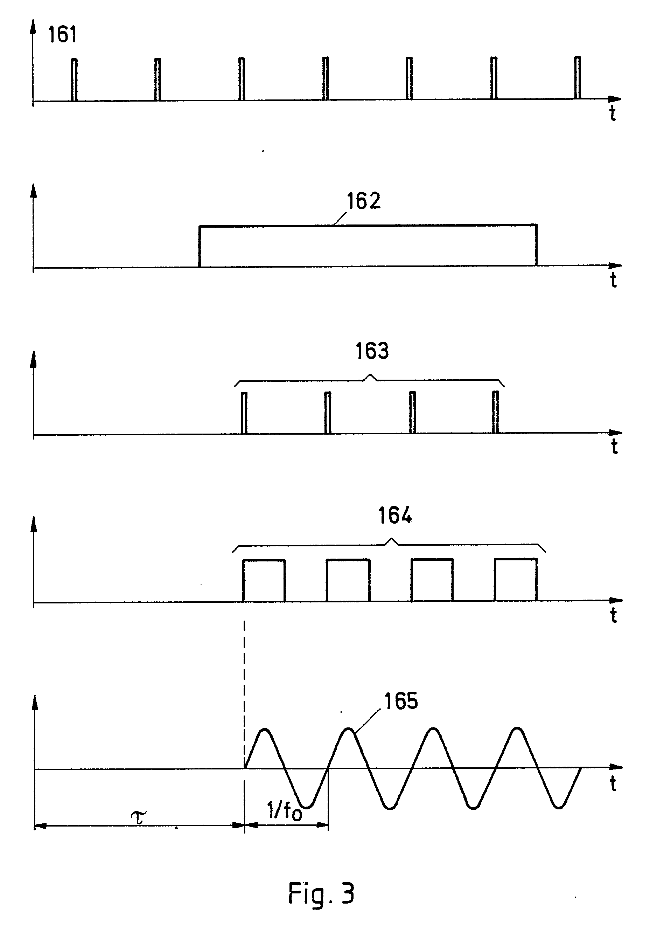

Das erfindungsgemässe Verfahren zur Erzeugung des Sendeimpulses für ein Wandlerelement der Ultraschallwandleranordnung wird anhand der Fig. 2 und 3 erläutert. Wie oben bereits erwähnt, müssen die Sendeimpulse eine für jedes Wandlerelement und jeden Richtungswinkel vorher bestimmte zeitliche Lage besitzen. Diese zeitliche Lage des Sendeimpulses wird auf folgende Weise erzeugt:

An jedem einer Mehrzahl von Eingängen 148 eines ersten Multiplexers 146 ist eine Impulsfolge der Sendefrequenz fo (wie 161 in Fig. 3) vorhanden. Die Phasenwinkel dieser Impulsfolgen sind in Schritten von einem Bruchteil einer Periode zwischen 0 und 360° gestaffelt, z.B. in Schritten von 45°.

Der Multiplexer 146 spricht auf eine ihm über eine Leitung 144 zugeführtes Steuersignal an, um eine der an seinen Eingängen vorhandenen Impulsfolgen über eine Leitung 151 weiterzuleiten.

An jedem einer Mehrzahl von Eingängen 147 eines zweiten Multiplexers 145 ist ein Impuls (wie 162 in Fig. 3) vorhanden. Die zeitlichen Lagen dieser Impulse sind ebenfalls in gleichen Intervallen von z.B. einer Periode der Sendefrequenz gestaffelt. Die Dauer von jedem dieser Impulse 162 umfasst mehrere Impulse einer Impulsfolge 161 (siehe Fig. 3). Der zweite Multiplexer 145 spricht auf ein ihm über eine Leitung 143 zugeführtes Steuersignal an, um einen der an seinen Eingängen vorhandenen Impulse über eine Leitung 149 weiterzuleiten.

Über eine Leitung 141 und ein Datenregister 142 steuert eine Recheneinheit 73 die Funktion des ersten und zweiten Multiplexers. Mit dem über die Leitung 144 dem ersten Multiplexer 146 zugeführten Steuersignal wird die Impulsfolge ausgewählt, die zur Erzeugung des Sendesignals den geeigneten Phasenwinkel hat und die deshalb vom ersten Multiplexer 146 durchgelassen wird.

Mit dem über die Leitung 143 dem zweiten Multiplexer 145 zugeführten Steuersignal wird der Impuls gewählt, der zur Erzeugung des Sendesignals die geeignete zeitliche Lage hat und der deshalb vom zweiten Multiplexer durchgelassen wird.

Mit einem Multiplexer 152, der z.B. ein UND-Gatter sein kann, wird das Produkt der Impulsfolge 161, die einem ersten Eingang des Multiplizierers über die Leitung 151 zugeführt wird, mit dem Impuls 162 gebildet, der einem zweiten Eingang des Multiplizierers über die Leitung 149 zugeführt wird. Das Produktsignal 163 ist eine Anzahl Impulse der Sendefrequenz, wie aus Fig. 3 ersichtlich. Aus diesem Produktsignal werden mit einer Impulsformstufe 153 zunächst einige Impulse 164 und daraus ein Sendeimpuls 165 abgeleitet, der dem Wandlerelement über eine Leitung 154 zugeführt wird. Die jeweils gewünschte zeitliche Lage des Sendeimpulses, die z.B. durch eine Verzögerung T in Bezug auf ein Referenzsignal definiert werden kann, wird also dadurch hergestellt, dass man eine Impulsfolge 161 geeigneter Phase mit einem Impuls 162 geeigneter zeitlicher Lage multipliziert und dass man den Sendeimpuls aus dem Produktsignal ableitet.

Aus Fig. 3 ist es ersichtlich, dass man das Produktsignal 163 allein durch die Wahl der zeitlichen Lage der Impulsfolge 161 um ± ½ Periode der Sendefrequenz verschieben kann, wobei der verwendete Impuls 162 unverändert bleibt. Für grössere Verschiebungen muss ein anderer Impuls 162 geeigneter Lage verwendet werden.

Die Berechnung des geeigneten Phasenwinkels der Impulsfolge 161 bzw. der geeigneten zeitlichen Lage des Impulses 162, die jeweils durch eine Verzögerung in Bezug auf ein Referenzsignal definiert werden können, wird in der Recheneinheit 73 durchgeführt.

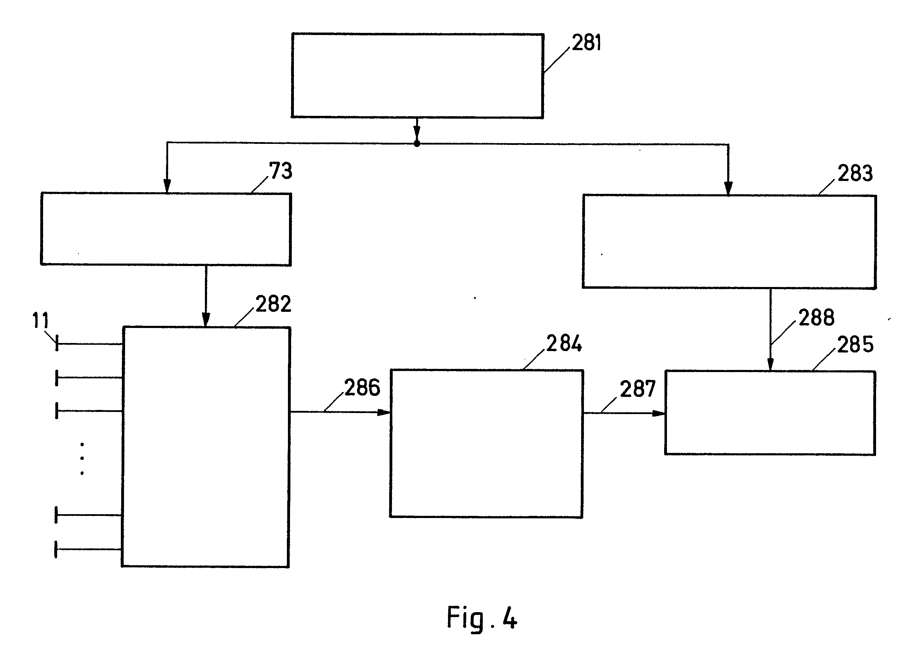

Die Verwendung der erfindungsgemässen Vorrichtung in einem Ultraschall-Abbildungsgerät, das z.B. zur Untersuchung des Herzens dient, wird nun anhand der Fig. 4 erläutert.

Die gesamte Anordnung der erfindungsgemässen Schaltungen, mit denen die Sendesignale für die Wandlerelemente 11 erzeugt werden, und der Schaltungen, mit denen die Echosignale verzögert werden, ist in Fig. 4 durch den Block 282 dargestellt. Dieser Block wird von der Recheneinheit 73 gesteuert.

Die von der Anordnung 282 abgegebenen, verzögerten Echosignale werden einem Echosignalempfänger 284 über eine Leitung 286 zugeführt. In diesem Empfänger werden die Echosignale in an sich dem Fachmann bekannter Weise verarbeitet.

Die verarbeiteten Echosignale werden einem geeigneten Anzeigegerät 285, z.B. einem Kathodenoszillograph zugeführt. In diesem Fall werden die Echosignale zur Steuerung der Intensität des elektronischen Strahls im K.O. dessen Z-Eingang zugeführt. Die Eingänge x und y des K.O. werden von einer dem Fachmann ebenfalls bekannten Monitorsteuerung 283 gesteuert, um eine geeignete Darstellung der durch die Abtastung des untersuchten Körpers mit dem Ultraschallstrahl gewonnenen Ultraschall-Abbildung zu erzeugen.

Die Recheneinheit 73 und die Monitorsteuerung 283 erhalten von einer zentralen Steuereinheit 281, die z.B. einen Zähler enthält, ein Signal, das der jeweiligen Strahlnummer entspricht, wobei diese Nummer den Richtungswinkel der gesendeten bzw. empfangenen Ultraschallwellen definiert. Die zentrale Steuereinheit trägt also dazu bei, dass zwischen der Abtastung des untersuchten Körpers mit dem Ultraschallstrahl und der Darstellung dieser Abtastung mit dem Anzeigegerät eine geeignete zeitliche Beziehung hergestellt wird.

## Patentansprüche

1. Ultraschall-Bildgerät mit einer Wandlereinrichtung, die aus einer fester länglichen Reihe Wandlerelemente (11) besteht und mit der ein Ultraschallstrahl (12) durch Umwandlung impulsartiger, von einem Sender (232) abgegebener Sendesignale erzeugt wird, wobei jedem Wandlerelement (11) ein Sendesignal mit einer vorher bestimmten zeitlichen Lage zugeführt wird, dadurch gekennzeichnet, dass der Sender enthält:
a) einen Multiplizierer (152) zur Bildung des Produkts aus einer Impulsfolge (161) der Sendefrequenz und einem Impuls (162);
b) einen ersten mit einem Eingang des Multiplizierers verbundenen Multiplexer (146), an dessen Eingängen (148) eine Mehrzahl von gegeneinander um einen Bruchteil einer Periode des Sendesignals verschobenen Impulsfolgen der Sendefrequenz und vorbestimmter Phasenwinkel vorhanden sind und der auf ein erstes Steuersignal (144) anspricht, um einen ersten Eingang des Multiplizierers wahlweise eine der Impulsfolgen zuzuführen, die einen geeigneten Phasenwinkel hat;
c) einen zweiten mit einem zweiten Eingang des Multiplizierers verbundenen Multiplexer (145), an dessen Eingängen (147) eine Mehrzahl von Impulsen gelangen, die in Bezug auf ein Referenzsignal verschiedene Verzögerungen aufweisen und deren Dauer mehrere Impulse einer Impulsfolge der Sendefrequenz umfasst, welcher zweite Multiplexer auf ein zweites Steuersignal (143) anspricht, um einem zweiten Eingang des Multiplizierers wahlweise einen der Impulse zuzuführen, der eine geeignete Verzögerung in Bezug auf das Referenzsignal hat, und
d) einen mit dem Ausgang des Multiplizierers verbundenen Signalformer (153), der dazu dient, von dem Ausgangssignal des Multiplizierers einen eine Sinuswelle der Sendefrequenz einhüllenden Sendeimpuls (165) abzuleiten, der einem Wandlerelement (11) der Ultraschallwandleranordnung zugeführt wird.

2. Ultraschall-Bildgerät nach Anspruch 1, dadurch gekennzeichnet, dass der Multiplizierer einen UND-Gatter enthält.

3. Ultraschall-Bildgerät nach Anspruch 1, dadurch gekennzeichnet, dass die Sendeschaltung eine über ein Datenregister (142) mit den beiden Multiplexern verbundene Recheneinheit (73) enthält, die dazu dient, für jedes Wandlerelement und für jeden Richtungswinkel den geeigneten Phasenwinkel der Impulsfolge und die geeignete Verzögerung des Impulses zu berechnen und dem ersten und dem zweiten Multiplexer Steuersignale abzugeben, die den berechneten Werten entsprechen.

4. Verwendung des Ultraschall-Bildgerätes nach Anspruch 1 für die medizinische Ultraschall-Diagnostik.

## Claims

1. An ultrasonic imaging apparatus comprising a transducer device made up of a fixed elongate row of transducer elements (11) and apt to be activated for transmitting an ultrasonic beam (12) by converting pulsed signals sent from a transmitter (232), each transducer element (11) being supplied with a transmitter signal having a predetermined position in time, characterized in that the transmitter contains:
a) A multiplier (152) for obtaining the product of a pulse (162) by a pulse sequence (161) at the transmitter frequency;
b) A first multiplexer (146) connected to one input of the multiplier, the multiplexer inputs (148) being supplied with a plurality of pulse sequences at the transmitter frequency at a predetermined phase angle and offset from one another by a fraction of a period of the transmitted signal, the multiplexer responding to a first control signal (144) for supplying a first input of the multiplier with a selected pulse sequence having a suitable phase angle;
c) A second multiplexer (145) connected to a second multiplier input, the multiplexer inputs (147) being supplied with a plurality of pulses which are delayed by varying amounts relative to a reference signal and the duration of which covers a number of pulses of a pulse sequence at the transmitted frequency, the second multiplexer responding to a second control signal (143) for supplying a second multiplier input with a chosen pulse having a suitable delay relative to the reference signal, and
d) A signal-shaping device (153) connected to the multiplier output and adapted to derive from the multiplier output signal a transmitting pulse (165) containing a sine wave at the transmitted frequency, the pulse being supplied to a component (11) of the ultrasonic converter system.

2. An ultrasonic imaging apparatus according to claim 1, characterized in that the multiplier contains an AND gate.

4

3. An ultrasonic imaging apparatus according to claim 1, characterized in that the transmitter circuit comprises a computer unit (73) connected via a data register (142) to the two multipliers and adapted to calculate the appropriate phase angle of the pulse sequence and the appropriate delay of the pulse for each converter component and each directional angle and to supply the first and second multiplier with control signals corresponding to the calculated values.

4. Use of the ultrasonic imaging apparatus according to claim 1 for ultrasonic medical diagnosis.

**Revendications**

1. Appareil de visualisation par ultrasons comportant un dispositif transducteur qui se compose d'une rangée oblongue rigide d'éléments transducteurs (11) et avec lequel on produit un faisceau ultrasonore (12) par transformation de signaux impulsionnels émis par un émetteur (232), un signal d'émission dont la position temporelle est préalablement déterminée étant appliqué à chaque élément transducteur (11), caractérisée en ce que l'émetteur contient:

a) un multiplieur (152) pour la formation du produit d'une suite d'impulsions (161) de la fréquence d'émission et d'une impulsion (162);

b) un premier multiplexeur (146) relié à une entrée du multiplieur, multiplexeur aux entrées (148) duquel se trouvent une pluralité de suites d'impulsions à la fréquence d'émission et d'une phase prédéterminée, décalées les unes par rapport aux autres d'une fraction de période du signal d'émission, et qui répond à un premier signal de commande (144), pour amener à une première entrée du multiplieur au choix l'une des suites d'impulsions, qui possède une phase appropriée;

c) un deuxième multiplexeur (145) relié à une deuxième entrée du multiplieur, et aux entrées (147) duquel aboutissent une pluralité d'impulsions, qui présentent différents retards par rapport à un signal de référence, et dont la durée comprend plusieurs impulsions d'une suite d'impulsions de la fréquence d'émission, second multiplexeur qui répond à un second signal de commande (143), pour conduire à une seconde entrée du multiplieur au choix l'une des impulsions, qui possède un retard approprié par rapport au signal de référence; et

d) un formateur de signal (153) relié à la sortie du multiplieur et qui sert à dériver du signal de sortie du multiplieur une impulsion d'émission (165), contenant une onde sinusoïdale de la fréquence d'émission, qui est amenée à un élément transducteur (11) du dispositif transducteur à ultrasons.

2. Appareil selon la revendication 1, caractérisée en ce que le multiplieur contient une porte ET.

3. Appareil selon la revendication 1, caractérisée en ce que le circuit d'émission contient une unité de calcul (73) reliée aux deux multiplexeurs par l'intermédiaire d'un registre de données (142), unité qui sert à calculer, pour chaque élément transducteur et pour chaque angle de direction, la phase appropriée de la suite d'impulsions et le retard approprié de l'impulsion et pour donner au premier et au second multiplexeurs les signaux de commande qui correspondent aux valeurs calculées.

4. Application de l'appareil selon la revendication 1 au diagnostic médical par ultrasons.

Fig. 1

Fig. 2

Fig. 3

Fig. 4